# EUROPÄISCHE PATENTSCHRIFT

(11) **EP 3 406 199 B1**
(45) Veröffentlichungstag und Bekanntmachung des Hinweises auf die Patenterteilung: **25.06.2025**
(21) Anmeldenummer: 18174071.3
(22) Anmeldetag: 24.05.2018
(51) Int. Cl.: A61B 10/00, G01K 13/00

(54) **NETZUNABHÄNGIGES FUNKTHERMOMETER FÜR DEN LANGZEITEINSATZ**
OFF-LINE RADIO THERMOMETER FOR LONG-TERM USE
THERMOMÈTRE RADIO INDÉPENDANT DU RÉSEAU POUR UNE UTILISATION À LONG TERME

(30) Priorität: 24.05.2017 DE 102017208883; 29.09.2017 DE 102017217477
(43) Veröffentlichungstag der Anmeldung: 28.11.2018
(73) Patentinhaber: bel'apps GmbH, 46045 Oberhausen (DE)
(72) Erfinder: Martin, Thomas, 38640 Goslar (DE)
(74) Vertreter: FARAGO Patentanwälte GmbH

(56) Entgegenhaltungen:
- WO-A1-2017/015661
- CA-A1- 2 886 522
- DE-A1- 102009 002 906
- US-A1- 2012 016 258
- US-A1- 2014 121 557
- US-A1- 2016 213 354
- US-A1- 2016 270 768
- US-A1- 2016 331 244

## Beschreibung

### Gebiet der Erfindung

Die Erfindung betrifft ein netzunabhängiges Funkthermometer zur Temperaturmessung in einer Körperöffnung für den Langzeiteinsatz und ein Verfahren zur Messung der Basaltemperatur über einen längeren Zeitraum unter Zuhilfenahme des erfindungsgemäßen netzunabhängigen Funkthermometers.

### Hintergrund der Erfindung

Es ist bekannt, dass sich im Verlauf des weiblichen Zyklus die Körpertemperatur hormonbedingt ändert. Man unterscheidet zwischen "Tieflage" (Temperaturniveau zwischen Menstruationsende und Eisprung) und "Hochlage" (Temperaturniveau zwischen Eisprung und Menstruationsbeginn). Während der Hochlage liegt das Temperaturniveau etwa 0,3° - 0,6°C über der Tieflage. Der Wechsel von Tieflage zu Hochlage kann 72 Stunden dauern - während dieser Zeit findet der Eisprung statt. Die weibliche Eizelle kann ab diesem Zeitpunkt für ca. 24 Stunden befruchtet werden.

Je genauer man den Zeitpunkt des Eisprungs bestimmen kann, umso sicherer kann eine Befruchtung erreicht bzw. vermieden werden. Nach Knaus-Ogino soll die Körpertemperatur der Frau regelmäßig mit einem Thermometer gemessen werden. Durch regelmäßige Messung und Aufzeichnung der Körpertemperatur kann dann eine Aussage über den Ovulationszeitpunkt getroffen werden.

Als grundsätzliches Problem erweist sich die Tatsache, dass die menschliche Körpertemperatur (auch ohne pathologische Indikationen) bereits im Allgemeinen im Tagesverlauf Schwankungen zeigt, z.B. in Abhängigkeit der jeweiligen körperlichen Aktivität. Die Amplitude dieser Schwankungen liegt mit 1,0 °C deutlich über dem Temperaturunterschied zwischen Tief- und Hochlage. Ferner ist zur Kenntnis zu nehmen, dass sich an verschiedenen Körperregionen zeitgleich gemessene Temperaturen sowohl um mehrere Grad Celsius als auch in der Wiederholgenauigkeit unterscheiden.

Man unterscheidet z.B. zwischen Oberflächentemperatur (Achsel, Stirn, Handgelenk) und Körperkerntemperatur (rektal, vaginal).

Zur möglichst zuverlässigen Erkennung des Wechsels von Tieflage zu Hochlage muss daher die Körperkerntemperatur gemessen werden und zwar vorzugsweise zu einem Zeitpunkt, wo erwartet werden darf, dass sie sich, unbeeinflusst von körperlichen Aktivitäten, auf einem stabilen und reproduzierbaren Niveau befindet. Diese Temperatur wird als "Basaltemperatur" bezeichnet und entspricht dem Temperaturminimum (Basis), das sich während des Schlafens im Körperkern einstellt.

Traditionell wird bei der Temperaturmethode die Körperkerntemperatur allmorgendlich am Ende der Nachtruhe, die per Wecker zu jeweils demselben Zeitpunkt abgebrochen werden muss, per Fieberthermometer gemessen und per Millimeterpapier als Temperaturgraph dokumentiert.

Während der Temperatursprung von Tief- zu Hochlage den Ovulationszeitpunkt sehr zuverlässig anzeigt, birgt das oben geschilderte traditionelle Prozedere eine Vielzahl von Fehlerquellen bei der Ermittlung dieses Temperatursprungs:
1. Mit der allmorgendlichen Messung nach dem Wecken wird nicht die eigentliche Basaltemperatur gemessen, die sich nur nach einigen Stunden Schlaf, d.h. ohne jegliche körperliche Aktivität einstellt. Während der Aufwachphase steigt die Körperkerntemperatur tendenziell wieder an, besonders, wenn das Aufwecken per Wecker geschieht und körperliche Aktivität erforderlich ist (Aufrichten im Bett, Licht einschalten, Thermometer vom Nachttisch holen, Thermometer einführen) um die Messung überhaupt durchführen zu können.
2. Die Messperson muss während des Messens absolut ungestört sein. Das Messergebnis wird mit zusätzlicher Ablenkung während der Messung immer ungenauer.
3. Auch bei rektaler oder vaginaler Messung mit einem handelsüblichen Thermometer besteht die Gefahr, dass sich dessen Position während der Messung verschiebt, ggfs. während erneuten Einschlafens aus dem Rektum oder der Vagina gleitet.
4. Es können Fehler beim Dokumentieren der Messwerte auf das Millimeterpapier passieren.
5. Es können Fehler beim Auswerten der Messkurve entstehen.

Des Weiteren ist die Anwendung dieser Methode recht unbequem, erfordert ein hohes Maß an Sorgfalt und muss sehr diszipliniert in den morgendlichen Tagesablauf eingebaut werden, damit brauchbare Ergebnisse entstehen können.

Aus dem Stand der Technik sind sogenannte "Ovulationscomputer" oder "Ovulationsthermometer" bekannt, die dieses Prozedere etwas vereinfachen.

Üblicherweise handelt es sich dabei um elektronische Thermometer mit, je nach Ausstattung, eingebautem Wecker, der an das Messen erinnert, einem Speicher für mehrere Temperaturwerte, einer Auswertesoftware zur Bestimmung des Ovulationszeitpunktes, einer Anzeige für aktuelle und ältere Temperaturwerte oder des berechneten Ovulationszeitpunktes. Diese Ovulationsthermometer verhindern zwar Fehler bei der Dokumentation und Auswertung der Temperaturmesswerte, die oben geschilderten Probleme (siehe Punkte 1 bis 3) bei der Ermittlung der Basaltemperatur bleiben aber bestehen.

Als Verbesserung werden hier intravaginale Thermometer, wie beispielsweise in der EP 2 567 680 B1 offenbart, vorgeschlagen, die während der gesamten Nachtruhe intravaginal getragen werden und während dieser Zeit in bestimmten Intervallen (z.B. alle 5 Minuten) die Körperkerntemperatur messen und speichern. Typischerweise werden die aufgezeichneten Messreihen dieser Sensoren mittels eines besonderen Auslesegerätes ausgelesen. In manchen Fällen übernimmt dieses Auslesegerät dann gleichzeitig die Auswertung der Messreihen zur Bestimmung der realen Basaltemperatur sowie des Ovulationszeitpunktes. In anderen Fällen muss das Auslesegerät mit einem weiteren Computer verbunden werden, der die Auswertung übernimmt und die Ergebnisse anzeigt.

Typischerweise werden die Temperatursensoren mit Akkus betrieben, die dann beispielweise vom Auslesegerät oder einem Netzteil regelmäßig aufgeladen werden müssen, damit garantiert ist, dass der Sensor die nächste nächtliche Messreihe aufzeichnen kann.

Diese intravaginalen Thermometer haben noch Nachteile in Hinblick auf Komfort, Usability und Alltagstauglichkeit. So ist das Ovulationsthermometer der EP 2 567 680 B1 als Pessar ausgestaltet und muss in aufwändiger Weise am äußeren Muttermund platziert werden.

Die Notwendigkeit von Zusatzgeräten zum Auslesen der Messwerte verteuert die vorgenannten Lösungen. Zudem müssen hier teilweise die Zusatzgeräte per USB-Kabel mit Computern verbunden werden, auf denen zuvor entsprechende Treiber- und Anwendungssoftware installiert werden muss. Teilweise ermöglicht erst eine Internetseite des Anbieters das Betrachten der eigenen Messwerte auf einem Smartphone oder nur mit einer einzigen Smartphone Modellreihe.

Bedeutende Nachteile bestehen darin, dass die Mobilität dieser Systeme durch die Notwendigkeit von Zusatzgeräten sehr begrenzt ist und dass die Sensoren typischerweise nur eine Lebensdauer von nur 1 Jahr besitzen.

Die US 2016/213354 A1 beschreibt ein Funkthermometer gemäss des Oberbegriffs des Anspruchs 1, zur Temperaturmessung in einer Körperöffnung für den Langzeiteinsatz aufweisend eine Leiterplatte, einen Temperatursensor, eine Kommunikationseinheit befähigt zur Bluetooth-Übertragung und einen Datenspeicher. Die Aktivierung dieses Funkthermometers ist durch Temperaturänderungen induzierbar.

Das Funkthermometer der US 2016/213354 A1 ist dazu in der Lage Temperaturvariationen zu erfassen und aufzunehmen, jedoch werden diese als distinkte Temperaturwerte abgespeichert, wodurch ein hoher Speicherplatzbedarf entsteht. Dies bedingt einen hohen Übertragungsbedarf und somit einen hohen Energiebedarf.

Es ist eine Aufgabe der vorliegenden Erfindung, ein Ovulationsthermometer zur Verfügung zu stellen, das hinsichtlich der oben aufgeführten Nachteile verbessert ist.

### Zusammenfassung der Erfindung

In einem ersten Aspekt wird diese Aufgabe gemäß der Erfindung dadurch gelöst, dass ein netzunabhängiges Funkthermometer zur Temperaturmessung in einer Körperöffnung nach Anspruch 1 bereitgestellt wird.

Durch Ausgestaltung als netzunabhängiges Funkthermometer liegt eine autark arbeitende Messeinheit vor, die kein Aufladen mit einem entsprechenden Ladegerät benötigt. Dadurch wird das Funkthermometer besonders benutzerfreundlich, und auch weniger fehleranfällig.

Da für das Funkthermometer auch kein Batterie- oder Akkuwechsel unter Öffnung des Thermometergehäuses erforderlich ist, kann es fabrikseitig als hermetisch verschlossenes System bereitgestellt werden und so den hohen hygienischen Anforderungen für eine Messung in Körperöffnungen gerecht werden.

Durch eine Reihe technischer Merkmale ist es den Erfindern gelungen, ein Thermometer für den Langzeiteinsatz bereitzustellen. Unter einem Langzeiteinsatz gemäß der Erfindung ist ein Einsatz über mindestens ein Jahr, bevorzugt mindestens zwei Jahre und besonders bevorzugt über drei, vier oder sogar fünf Jahre bei täglicher Messung zu verstehen.

So erlaubt die Verwendung eines Halbleiter-Temperatursensors eine genaue Messung bei geringem Energieverbrauch. Dieser Sensortyp weist zudem eine hohe Langzeitstabilität hinsichtlich der Temperaturmessung auf.

Durch Verwendung eines besonderen Halbleiter-Temperatursensors mit innovativer Signalprozessierung und innovativem analogem Design weist der Halbleiter-Temperatursensor eine hohe Auflösung sowohl über einen großen Temperaturbereich als auch über einen großen Spannungsbereich auf.

Erfindungsgemäß werden die Inkremente oder Dekremente nicht auf den Basiswert (Offset-Wert) bezogen, sondern auf den jeweils vorangehenden Wert. Da die Temperaturdifferenz von aufeinanderfolgenden Messzeitpunkten sehr gering oder sogar Null ist, sind die hierbei erfassten Messwerte deutlich kleiner, so dass die Länge der Messreihen um weitere 50 bis 62.5% verkürzt werden kann.

Stromsparend ist auch die Signalübertragung mittels Bluetooth Smart nach dem BLE-Standard.

Durch die Steuerbarkeit des Funkthermometers über die Temperatur wird nicht nur eine autarke, von der Benutzersteuerung unabhängige und funktionsoptimierte Aktivierung ermöglicht, sondern diese kontrollierte Betriebssteuerung trägt auch zur Minimierung des Stromverbrauchs bei.

Die Erfassung, Abspeicherung und Weitergabe von inkrementellen oder dekrementellen Temperaturdaten reduziert in entscheidender Weise das Datenvolumen, so dass nicht nur längere Messreihen abgespeichert werden können, sondern auch die Übertragungszeit der energieintensiven Funkübertragung reduziert werden kann.

Dies alles ermöglicht es, mit einer handelsüblichen Knopfzellenbatterie ein Funkthermometer bereitzustellen, das über mehrere Jahre dauerhaft mit hoher Präzision und Messstabilität eingesetzt werden kann.

Durch die Verwendung der Bluetooth Smart-Technologie kann das Funkthermometer mit zahlreichen Empfangsgeräten kommunizieren. Hier ist insbesondere die Verwendung mit einem Smartphone oder Tablet möglich, bei dem eine Anwendungssoftware, als sogenannte "App" verwendet werden kann. Diese Art der Auswertung findet gerade bei dem betreffenden jugendlichen Anwenderkreis eine hohe Akzeptanz.

Damit wird es erstmals möglich, die Temperaturmessmethode nach Knaus-Ogino bei geringen Kosten so zu vereinfachen, dass sie einer breiten Anwendung zugänglich ist.

### Die Erfindung im Einzelnen

Erfindungsgemäß besitzt das Funkthermometer die Eignung, die Temperaturmesswerte als Inkremente oder Dekremente gegenüber einer Basistemperatur abzuspeichern und dann auch mittels Bluetooth zu übertragen. Dies ist möglich, da nur Messwerte zwischen 35,5°C und ca. 40°C relevant sind und daher die Inkrementwerte deutlich kleiner sind als die Absolutwerte der Messung. In einer bevorzugten Ausführungsform wird hierbei die Temperatur von 35,5°C als Basiswert oder Offset-Wert genommen und alle weiteren Werte als Differenz zu diesem Wert ausgedrückt. Der Basiswert muss einerseits so hoch sein, dass er physiologisch relevant ist, aber auch so niedrig, dass nur höhere Werte als Inkremente und keine niedrigeren Werte als Dekremente zu erwarten sind. Durch die Inkrement- oder Dekrementspeicherung und -übermittlung verkürzt sich die Länge der Messreihen um ca. 50%.

In einer bevorzugten Ausführungsform sind die elektronischen Komponenten auf einer, bevorzugt beidseitig bestückten, ersten Leiterplatte angebracht, wobei eine der Kanten der ersten Leiterplatte als leicht gebogene metallisierte Kante direkt mit dem Minuspol der Knopfzellenbatterie in Kontakt steht, und wobei die Verbindung zum Pluspol der Batterie über eine U-förmige Batteriekontaktfeder hergestellt wird, deren mittlere Basis bevorzugt in eine kantenseitig metallisierte Aussparung der Leiterplatte eingreift.

Erfindungsgemäß besitzt das Funkthermometer eine Knopfzellenbatterie. Diese ist bevorzugterweise eine Lithiumbatterie.

In einer weiteren Ausführungsform weist die Batterie eine Leistung von zwischen 0,1 und 2 Watt, bevorzugt von zwischen 0,2 und 1 Watt und besonders bevorzugt von ca. 0,5 Watt auf.

Das erfindungsgemäße netzunabhängige Funkthermometer (im Folgenden auch als "Funkthermometer" bezeichnet) kann in unterschiedlichste Körperöffnungen eingeführt werden und dort zur Temperaturmessung verwendet werden. Beispielhaft seien hier der Mundraum, die Achselhöhle, das Rektum oder die Vagina genannt.

In einer bevorzugten Ausführungsform wird das Funkthermometer zur Messung der Basaltemperatur verwendet und kann dementsprechend bevorzugt im Rektum und vor allem in der Vagina eingesetzt werden.

Erfindungsgemäß ist das Funkthermometer für einen Langzeiteinsatz ausgelegt. Der "Langzeiteinsatz" ist erfindungsgemäß als ein Einsatz definiert, bei dem das Funkthermometer bei täglicher Erfassung einer Messreihe über mindestens 6 Stunden und täglicher Übermittlung der Daten einen Einsatz über einen Zeitraum von mindestens einem Jahr, bevorzugt mindesten zwei Jahren und besonders bevorzugt von mindestens drei, vier oder sogar fünf Jahren ermöglicht.

In einer Ausführungsform der Erfindung besteht die Kunststoffkapsel des Funkthermometers im Wesentlichen aus einem Kunststoff, der eine Körperverträglichkeit (Biokompatibilität) gemäß **DIN** ISO 10993-5:2009 aufweist. Unter einer "im wesentlichen" vorhandenen Ausgestaltung mit diesem Kunststoff ist im Kontext der Erfindung eine Ausgestaltung zu verstehen, bei der zumindest die äußere, dem Körper zugängliche Oberfläche aus diesem Kunststoff besteht.

In bevorzugter Weise ist der biokompatible Kunststoff hierbei ausgewählt aus der der Gruppe aufweisend Polycarbonat, Polyacrylat, Polyester, Polyethylen, Polypropylen, Polyacrylnitril, Acrylnitril-Butadien-Styrol-Copolymere (ABS) und Gemische hiervon.

Bei dem ABS-Polymer variieren die Mengenverhältnisse der eingesetzten Monomere von 15-35 % Acrylnitril, 5-30 % Butadien und 40-60 % Styrol.

In besonders bevorzugter Weise besteht die Kapsel aus einem Gemisch aus 80% ABS-Polymer, 10% Polycarbonat und 10% Kautschuk.

Als hermetisch verschlossene Struktur lässt sich die Kunststoffkapsel in einfacher Weise mit Wasser und/oder flüssigen Desinfektionsmitteln reinigen.

In einer Ausführungsform der Erfindung ist die Kunststoffkapsel des Funkthermometers ein über die Hälfte seiner Länge abgeflachter Kreiszylinder, der an seinem zylindrischen Ende bevorzugt halbkugelförmig abgerundet ist und an seinem abgeflachten Ende eine Öse zur Aufnahme einer Zugvorrichtung aufweist. Damit ist das Funkthermometer einfach und benutzerfreundlich in die Körperöffnung einzuführen. Durch die unterschiedliche Ausgestaltung der beiden Enden wird eine Fehlorientierung beim Einführen in die Körperöffnung verhindert. Zudem kann durch die Öse in Verbindung mit einer Zugvorrichtung das Funkthermometer wieder einfach aus der Körperöffnung entnommen werden. Durch die Formannäherung an übliche Menstruationstampons ist der Benutzer mit der Anwendung und dem Tragegefühl des Funkthermometers auf Anhieb vertraut, was in einer erhöhten Compliance resultiert.

Als Zugvorrichtung kann hier zweckmäßigerweise ein Band oder eine Schnur verwendet werden. In bevorzugter Weise wird ein ringförmige Silikonschnur verwendet, das unter Ausbildung einer Schlaufe durch die Öse gezogen werden kann.

In einer Ausführungsform hat die Kapsel eine Länge von weniger als 60 mm, bevorzugt von weniger als 50 mm, besonders bevorzugt von zwischen 35 und 50 mm und insbesondere von 47 mm.

In einer weiteren Ausführungsform hat die Kapsel an ihrer dicksten Stelle einen Durchmesser von weniger als 25 mm, bevorzugt von weniger als 20 mm, besonders bevorzugt von zwischen 12 und 16 mm und insbesondere von 14 mm.

Das Funkthermometer besitzt zweckmäßigerweise ein Gesamtgewicht von weniger als 15 g, bevorzugt von weniger als 10 g, besonders bevorzugt ein Gesamtgewicht von zwischen 6 und 8 g und insbesondere von 7 g.

In einer weiteren Ausführungsform der Erfindung besteht bei dem Funkthermometer die Kunststoffkapsel aus einem Kapselgehäuse und einem Kapseldeckel. Es handelt sich somit hierbei aus einem einfachen, nur aus zwei Teilen bestehenden Kapselaufbau, was das Risiko von Undurchlässigkeiten reduziert und eine kostengünstige Lösung darstellt.

In bevorzugter Weise wird das Kapselgehäuse mit dem Kapseldeckel vor dem Einsatz als Thermometer durch Verklebung fest und wasserdicht miteinander verbunden. Die Verbindung dieser beiden Teile geschieht in geeigneter Weise fabrikseitig, so dass nur kontrolliert verschlossene, d.h. einer Qualitätskontrolle unterworfene, Funkthermometer zur Anwendung kommen.

In einer Ausführungsform wird für die Verklebung bevorzugt ein UV-härtender Cyanacrylatkleber eingesetzt. Diese stellen biokompatible Kleber mit hoher Klebfestigkeit dar.

In einer weiteren Ausführungsform der Erfindung weist der Halbleiter-Temperatursensor mindestens eine der folgenden Eigenschaften auf:
(a) Eine Auflösung von mindestens 0,05 °C und bevorzugt von 0,01 °C im Bereich der Körpertemperatur;
(b) Abgabe einer digital umgesetzten Spannung über eine serielle Schnittstelle, die bevorzugt eine I2 C-Schnittstelle ist;
(c) Eine Langzeitstabilität von weniger als 0,05°C/Jahr, bevorzugt von weniger als 0,01 °C/Jahr;
(d) Anwesenheit eines integrierten Mikrocontrollers, der die Messfehler des eigentlichen Halbleitersensors korrigiert;
(e) Fabrikseitige Kalibrierung mit Speicherung der Kalibrierdaten auf dem Temperatursensor-Chip in einem Permanentspeicher;
(f) ein durchschnittlicher Stromverbrauch von weniger als 200 nA pro Messung bei einer Messung pro Sekunde.

Eine Auflösung von 0,05 °C im Bereich der Körpertemperatur ist notwendig, um die Basaltemperatur zur Ovulationsmessung ausreichend genau messen zu können. **In** bevorzugter Weise hat der erfindungsgemäße Halbleiter-Temperatursensor eine Auflösung von 0,01 °C.

Mit einer Abgabe einer digital umgesetzten Spannung über eine serielle Schnittstelle, die bevorzugt eine I2 C-Schnittstelle ist, ist eine effiziente Datenübermittlung möglich.

Halbleitersensoren weisen eine hohe Langzeitstabilität auf. **In** bevorzugter Weise hat der Halbleitersensor eine Langzeitstabilität von weniger als 0,05°C/Jahr, bevorzugt von weniger als 0,01 °C/Jahr. Erst hierdurch ist eine sehr lange, das heißt über mehrere Jahre sich erstreckende Einsatzfähigkeit des Funkthermometers zur Messung der vaginalen Basaltemperatur gegeben. Der erfindungsgemäße Halbleiter-Temperatursensor hat den Vorteil, auch bei während des Langzeitbetriebs abnehmender Spannung seine Messfähigkeit und hier insbesondere seine Auflösung aufrechtzuerhalten. Der Halbleiter-Temperatursensor weist somit kein spannungsabhängiges Driften auf.

Durch Anwesenheit eines integrierten Mikrocontrollers, der die Messfehler des eigentlichen Halbleitersensors korrigiert, wird eine kontinuierlich hochpräzise Temperaturmessung ermöglicht.

Zudem trägt eine fabrikseitige Kalibrierung des Temperatursensors mit Speicherung der Kalibrierdaten auf dem Temperatursensor-Chip in einem Permanentspeicher zur Messgenauigkeit bei.

Durch den Einsatz eines entsprechenden miniaturisierten Halbleiter-Temperatursensors kann die Messung mit einem durchschnittlichen Stromverbrauch von weniger als 200 nA pro Messung (bei einer Messung pro Sekunde) erfolgen.

**In** einer bevorzugten Ausführungsform weist der Halbleiter-Temperatursensor (auch "Bandgap"-Temperatursensor genannt) alle der vorab aufgelisteten Merkmale (a) bis (f) auf.

Dem Fachmann sind Halbleitersensoren mit entsprechenden Eigenschaften bekannt. Hier wird beispielsweise auf die I2 C-Temperatursensoren der Firma Silicone Laboratories Inc. (Austin, Texas USA) verwiesen, die unter anderem den Hochpräzisionstemperatursensor Si7051 beinhalten, der in einer besonders bevorzugten Ausführungsform eingesetzt werden kann.

Zweckmäßigerweise liegen die elektronischen Bauteile des Funkthermometers, wie beispielsweise Datenspeicher, Bluetooth LE-Übertragungseinheit und Steuerungseinheit als Single Chip Computer("System-on-a-chip"; SOC) vor. Damit wird eine kostengünstige, platzsparende und stromsparende Ausgestaltung ermöglicht.

**In** einer bevorzugten Ausführungsform der Erfindung sind bei dem Funkthermometer die elektronischen Komponenten auf einer, bevorzugt beidseitig bestückten, ersten Leiterplatte angebracht, wobei eine der Kanten der ersten Leiterplatte über eine senkrecht dazu angebrachte zweite kreisrunden Leiterplatte mit dem Minuspol der Knopfzellenbatterie in Kontakt steht, und wobei die Verbindung zum Pluspol der Batterie über eine U-förmige Batteriekontaktfeder hergestellt wird, deren mittlere Basis bevorzugt zwischen erster und zweiter Leiterplatte angeordnet ist. Diese Ausgestaltung ist besonders platzsparend und ermöglicht erst eine Ausgestaltung als geringdimensionierte Tamponform.

In einer alternativen Ausführungsform sind bei dem Funkthermometer die elektronischen Komponenten auf einer, bevorzugt beidseitig bestückten, ersten Leiterplatte angebracht, wobei eine der Kanten der ersten Leiterplatte als leicht gebogene metallisierte Kante direkt mit dem Minuspol der Knopfzellenbatterie in Kontakt steht, und wobei die Verbindung zum Pluspol der Batterie über eine U-förmige Batteriekontaktfeder hergestellt wird, deren mittlere Basis bevorzugt in eine kantenseitig metallisierte Aussparung der Leiterplatte eingreift. Dies ist eine zusätzlich vereinfachte Ausgestaltung, bei der auf die zweite Leiterplatte verzichtet werden kann.

In einem zweiten Aspekt stellt die Erfindung ein Verfahren zur Messung der Basaltemperatur zur Ovulationsbestimmung bereit, wobei dieses Verfahren die folgenden Schritte umfasst:
(a) Bereitstellen eines erfindungsgemäßen Funkthermometers in einem Ruhemodus;
(b) Einführen des Funkthermometers in eine Körperöffnung einer Frau, bevorzugt in die Vagina;
(c) Aufnehmen einer Temperaturmessreihe, bevorzugt über Nacht;
(d) Speicherung der Messdaten in einem Speicher des Funkthermometers;
(e) Entnehmen des Funkthermometers aus der Körperöffnung;
(f) Übertragen der Temperaturmessdaten mittels Bluetooth Smart an ein Empfangsgerät, das bevorzugt ein Smartphone oder ein Tablet ist;
(g) optionale Reinigung des Funkthermometers und Lagerung außerhalb der Körperöffnung;
(h) Wiederholung der Schritte (b) bis (g), bevorzugt täglich, zur Erhebung einer Langzeitmessreihe und Ermittlung relevanter Ereignisse im Menstruationszyklus wie Ovulationszeitpunkt, empfängnisfreie Phase oder fruchtbare Phase.

Erfindungsgemäß wird das Funkthermometer temperaturabhängig gesteuert, wobei die Aktivierung des Funkthermometers ausgehend von einem Ruhezustand durch das Einbringen in die Körperöffnung und der dadurch hervorgerufenen Temperaturerhöhung auf einen physiologischen Wert induziert wird.

In bevorzugter Weise erfolgt die Detektion der Ruhephase durch ein Verfahren, bei dem das Funkthermometer im Ruhemodus einen zeitgesteuerten Zähler inkrementiert und nach einer vorab festgelegten Zählzeit, die bevorzugt zwischen 10 Minuten und 60 Minuten, besonders bevorzugt zwischen 15 und 45 Minuten und insbesondere bevorzugt 30 Minuten beträgt, eine energiesparende Temperaturmessung mit geringer Präzision durchgeführt wird, und das Messergebnis mit dem Schwellenwert von 35,5°C verglichen wird, wobei der Ruhemodus solange aufrechterhalten bleibt, wie eine konfigurierbare Anzahl von aufeinander folgenden Messwerten, die bevorzugt mindestens zwei aufeinander folgende Messwerte entspricht, nicht über 35,5°C liegen. Durch dieses Messverfahren wird in einfacher und energiesparender Weise der Ruhezustand bis zum Einführen in die Körperöffnung kontrolliert aufrechterhalten.

In einer alternativen Ausführungsform wird das Funkthermometer in einen "Tiefschlafmodus" mit extrem niedrigem Energieverbrauch versetzt. Anstelle des oben beschriebenen Zählers kann die energiesparende Messung zur Detektion einer Lage in der Körperhöhlung auch durch eine stromsparende externe Timerschaltung initiiert werden, die bei Erreichen der eingestellten Zeit das Funkthermometer für diese "Testmessung" reaktiviert.

In einer bevorzugten Ausführungsform ist diese externe Timerschaltung ein Systemzeitgeber mit einem integrierten MOSFET-Treiber und einem Verbrauch im nanoAmpere-Bereich. In einer speziellen Ausführungsform wird hier der TPL5110"Nano-power system timer for power gating" der Firma Texas Instruments (Dallas, TX, USA) verwendet.

Erfindungsgemäß wird das im Ruhemodus vorliegende Funkthermometer beim Einführen in die Körperöffnung aktiviert, insofern eine Temperaturmessreihe initiiert wird. In bevorzugter Weise führt das Funkthermometer hierbei zur Initialisierung der Messreihe bei einer konfigurierbaren, mindestens 2 betragenden Anzahl von aufeinanderfolgenden Messwerten über 35,5°C die folgenden drei Maßnahmen durch:
(a) Es reduziert das Messintervall auf das für die Messreihe vorgesehene Messintervall, mit einer konfigurierbaren Reduktion, hierbei bevorzugt von 30 Minuten während der Ruhephase auf 5 Minuten während der Messreihe;
(b) Es erhöht die Messauflösung auf eine Auflösung von mindestens 0,05°C, wobei hier die Erhöhung der Auflösung bis zu einer für den Sensor maximalen Auflösung bevorzugt ist,
(c) Es speichert die Messwerte als Messreihe, wobei die Temperaturwerte als Inkremente ausgehend von einer Offset-Temperatur gespeichert werden.

**In** einer weiteren Ausführungsform ist bei dem erfindungsgemäßen Verfahren die Speicherung der Messdaten in einem Ringpuffer implementiert, bei dem die ältesten Inhalte überschrieben werden, wenn der Puffer voll ist und weitere Elemente in den Ringpuffer abgelegt werden. Somit bleiben auch bei länger ausbleibender Datenübertragung die jüngeren Daten gesichert und können zur Auswertung herangezogen werden.

**In** einer bevorzugten Ausführungsform ist der Speicher so dimensioniert, dass er ca. 30 oder mehr Messreihen aufnehmen kann. Da das Funkthermometer nicht während der Menstruation getragen wird und auch nicht zwischen Eisprung und Menstruation, bedeutet dies, dass das Funkthermometer über einen Zeitraum von mehr als einem Monat Daten erheben kann, ohne dass eine Datenübermittlung an das Empfangsgerät stattfinden muss.

Die Temperatursteuerung des erfindungsgemäßen Funkthermometers beinhaltet auch die automatische Erkennung der Entnahme aus der Körperöffnung mit den beiden zentralen Folgen, dass 1.) die Messreihe abgeschlossen wird und 2.) eine Datenübertragung initiiert wird.

**In** einer bevorzugten Ausführungsform erfolgt dies durch ein Verfahren bei dem das Funkthermometer nach dessen Entnahme aus der Körperöffnung und einer damit einhergehenden Detektion einer konfigurierbaren, mindestens 2 betragenden Anzahl von aufeinanderfolgenden Messwerten unter 35,5°C die Messreihe abgeschlossen wird und der Bluetooth-Übertragungseinheit die Verfügbarkeit einer abgeschlossenen Messreihe signalisiert wird (sog. "Advertising").

In einer weiteren Ausführungsform erfolgt die Datenübertragung in einem zweistufigen Verfahren, bei dem die Gerätesuche zum Verbindungsaufbau nur in einer kurzen Zeitspanne mit hoher Sendeleistung erfolgt und dann iterativ nach einer Ruhepause mit verminderter oder ausbleibender Aktivität einen neuen Versuch startet.

In einer bevorzugten Ausführungsform der Erfindung führt die Bluetooth-Übertragungseinheit zur Übertragung einer abgeschlossenen Messreihe die folgenden Verfahrensschritte durch:
i. die Übertragungseinheit versucht, für eine kurze Zeitspanne von weniger als 1 min bevorzugt weniger als 30 sec und besonders bevorzugt für 10 sec mit hoher Sendeleistung, eine Verbindung mit einer Empfangseinheit aufzubauen;
ii. bei erfolgreichem Verbindungsaufbau überträgt die Verbindungseinheit die abgeschlossene(n) Messreihe(n) an die Empfangseinheit und die entsprechenden Daten werden aus dem Speicher des Funkthermometers gelöscht; oder
iii. bei erfolglosen Verbindungsaufbau wird für eine Zeitspanne von zwischen 30 s und 5 min, bevorzugt von zwischen 45 s und 2 min und insbesondere für 1 min die Sendeleistung der Übertragungseinheit reduziert oder ganz abgeschaltet,
iv. Iteration der Schritte i. und iii. mit einer konfigurierbaren Iterationsrate von bevorzugt zwischen 40 und 80 und besonders bevorzugt von 60;
v. bei nicht erfolgreichem Verbindungsaufbau nach Erreichen der konfigurierten Iterationsrate erfolgt das Abschalten der Sendeinheit bis zum Abschluss einer weiteren Messreihe.

In einer speziellen Ausführungsform aktiviert der Sensor Nach Abschluss einer Messreihe seinen Bluetooth Transmitter für 10 Sekunden, um der Smartphone App die Verfügbarkeit einer neuen Messreihe per " Bluetooth-Advertising" zu signalisieren. Das 10 sekündige Bluetooth-Advertising wird auch als "Advertising-Burst" bezeichnet.

Initiiert die Smartphone App innerhalb dieser 10 Sekunden keinen Verbindungsaufbau mit dem Sensor, erzeugt dieser weitere Advertising-Bursts in Intervallen von jeweils 1 Minute - insgesamt jedoch höchsten 60 Intervallen (also eine Stunde lang).

Die Anzahl der Advertising-Bursts sei im Folgenden "Z[AB]" genannt. Im vorab beschriebenen Szenario beträgt Z[AB] also "60".

Das Wiederholen der Advertising-Bursts bietet der Benutzerin so eine "Karenzzeit" von max. einer Stunde, während der sie entdecken kann, dass ihr SmartPhone sich nicht in Reichweite des Sensors befindet, oder nicht eingeschaltet ist, etc., und ggfs. für Abhilfe sorgen kann, so dass die aktuelle Messreihe doch noch zeitnah abgeliefert werden kann.

Bei jedem Advertising-Burst wird Z[AB] dekrementiert. Sollte es bei Ablauf der Karenzzeit (Z[AB] = 0) zu keiner Datenübertragung gekommen sein kommt es zu einem "Timeout". Bei einem "Timeout" terminiert der Sensor sein "Advertising-Burst" Engagement bis eine neue Messreihe vorliegt und damit Z[AB] wieder auf 60 gestellt wird.

Normalerweise (d.h.: wenn sich das eingeschaltete Smartphone mit aktivierter App in Bluetooth-Reichweite befindet) initiiert die Smartphone App den Verbindungsaufbau mit dem Sensor bereits innerhalb des ersten Advertising-Bursts.

Es kann dazu kommen, dass der Sensor mehrere Tage (oder Wochen) täglich benutzt wird, ohne die aufgezeichneten Messreihen abgeliefert werden können, etwa, weil sich für längere Zeit kein SmartPhone in Bluetooth-Reichweite befindet (z.B. Urlaub ohne SmartPhone, SmartPhone-Verlust, SmartPhone-Defekt, etc.).

In diesen Fällen finden nach jeder aufgenommenen Messreihe 60 vergebliche Advertising-Bursts statt. Bei einem 10-tägigen Urlaub wären dies also 600 Advertising-Bursts. Der Stromverbrauch pro Advertising-Bursts ist signifikant. Unter normalen Bedingungen reichen 600 Advertising-Bursts für 600 Tage (d.h. bei ca. 15 Messreihen/Monat > 3 Jahre).

Als Lösung für diese Fälle werden die folgenden zwei Ausführungsformen vorgeschlagen, die entweder separat eingesetzt werden oder miteinander kombiniert werden: In einer ersten Ausführungsform wird bei vergebliche Advertising-Bursts mit einer vorgegebenen Anzahl die Z[AB] reduziert. So kann beispielsweise statt einer "Karenzzeit" von 1 Stunde mit 60 Advertising-Bursts in Intervallen von 1 Minute die Advertising Bursts auf 10 reduziert werden.

Zusätzlich können hierbei die Intervalle von einem Advertising-Burst zum nächsten Advertising-Burst sukzessive verlängert werden, z.B. von 1 auf 2 auf 3 auf 5 auf 10 auf 20 auf 45 auf 90 auf 180 auf 360 Minuten. In diesem Beispiel ergibt sich bei 10 Advertising-Bursts (Z[AB] = 10) eine Gesamt-Karenzzeit von knapp 12 Stunden (714 Minuten = 1 + 2 + 3 + 5 + 10 + 20 + 45 + 90 + 180 + 360).

Geeigneterweise lassen sich bei dieser Ausführungsform sowohl Z[AB] als auch die Intervalle bedarfsorientiert optimieren.

In einer zweiten Ausführungsform wird der Timeout verändert, also die Anzahl an vergeblichen Advertising-Bursts, die dann zu einem Abbruch ("Timerout") der Advertising Burst-Reihe führt. Der Sensor pflegt einen Zähler für aufeinander folgende Timeouts (im Folgenden "Z[TO]" genannt). Die vorab beschriebene Anzahl von Advertising-Bursts ("Z[AB]") ist abhängig von Z[TO]: Liegt Z[TO] über einem bestimmten Schwellwert (z.B. 3 Timeouts in Folge), wird Z[AB] auf z.B. 3 gesetzt ("Urlaubsmodus") ansonsten auf 10 (s. oben). Nach jeder erfolgreich an das SmartPhone abgelieferten Messreihe setzt der Sensor Z[TO] auf "0" (NULL).

In einer weiteren Ausführungsform erhält bei dem erfindungsgemäßen Verfahren bei der Messreihe zur Angabe der jeweiligen Messzeitpunkte nur ein Messwert einen Zeitstempel und die übrigen Zeitpunkte werden durch Abspeicherung des Messintervalls definiert. Damit wird das Datenvolumen in entscheidender Weise reduziert, da nicht jeder Messwert mit einem Zeitstempel versehen werden muss.

Zur Bestimmung der echten Messzeit wird auch der Übertragungszeitpunkt mit einem Zeitstempel versehen. Hierdurch wird das Empfangsgerät durch Differenzbildung den tatsächlichen Beginn der Messreihe ermitteln.

Bei der Übertragung der Daten vom Funkthermometer auf die Empfangseinheit können noch weitere Daten übertragen werden. Diese umfassen beispielsweise:
- Batteriezustand;
- Sendesignalstärke;
- aktuelle Konfigurationsparameter des Funkthermometers;
- Firmware Version des Funkthermometers
- Universally Unique Identifier (UUID) des Funkthermometers

In einer weiteren Ausführungsform kann die Empfangseinheit auch, bevorzugt unter Rückgriff auf die Cloud, eine Aktualisierung der Software oder bestimmter Konfigurationsparameter vornehmen.

In einer Ausführungsform der Erfindung wird die Empfangseinheit durch Ablesen eines, dem individuellen Funkthermometer zugeordneten, Barcodes oder eines zweidimensionalen Codes zur Aufnahme von Messreihen von diesem Funkthermometer berechtigt.

In bevorzugter Weise wird hier ein 2D-Code verwendet, der besonders bevorzugt ein QR-Code ist. Ein solcher 2D-Code kann bei kleinen Maßen eine hohe Informationsdichte zur sicheren Geräte-Erkennung bereitstellen. Ein solcher 2D-Code kann auf dem Funkthermometer selber oder der Thermometerverpackung angebracht sein. Der 2D-Code, der bevorzugt ein QR-Code ist, enthält zweckmäßigerweise einen Schlüssel mit dessen Hilfe die Software der Empfangseinheit (bevorzugt eine "App") die Daten aus der Cloud abrufen kann, die sich dazu selbst automatisch beim Cloudserver authentifizieren muss.

In einer weiteren Ausführungsform werden die Messdaten von der Empfangseinheit in verschlüsselter Form in einer über das Internet zugänglichen Datenbank (sog. "Cloud") abgespeichert und können aus dieser Cloud wieder ausgelesen werden.

In einem weiteren Aspekt stellt die Erfindung ein netzunabhängiges Funkthermometer zur Temperaturmessung für den Langzeiteinsatz bereit, das Folgendes umfasst:
(a) ein Single-Chip-Computer (SOC);
(b) einen Halbleiter-Temperatursensor;
(c) eine Kommunikationseinheit zur Signalübertragung nach dem BLE-Standard;
(d) einen Datenspeicher;
(e) eine Knopfzellenbatterie;

wobei der Temperatursensor und die Kommunikationseinheit über die Temperatur steuerbar sind, und
wobei das Funkthermometer zu Erfassung, Speicherung und Weitergabe von inkrementellen oder dekrementellen Temperaturwerten ausgelegt ist.

In einem weiteren Aspekt stellt die Erfindung ein Verfahren zur Bestimmung der Basaltemperatur bereit, wobei das Verfahren die folgenden Schritte umfasst:
(a) Bereitstellen einer über Nacht während der Schlafphase aufgenommenen Temperaturmessreihe eines weiblichen menschlichen Körpers mit einem Messintervall von zwischen 2 und 10 Minuten, und bevorzugt von 5 Minuten, wobei diese Temperaturmessreihe bevorzugt mit einem erfindungsgemäßen Funkthermometer und/oder einem erfindungsgemäßen Verfahren erstellt worden ist;
(b) Ermittlung desjenigen Zeitbereichs von 15 bis 25 Minuten und bevorzugt von 20 Minuten in der Temperaturmessreihe aus Schritt (a), der sowohl 20 bis 90 Minuten vor dem durch das Aufwachen definierten Ende der Messreihe liegt als auch die geringste Temperaturamplitude als Differenz zwischen dem höchsten und niedrigsten in diesem Zeitbereich gemessenen Temperaturwerte aufweist;
(c) Bestimmung der Basaltemperatur durch Bildung des Mittelwerts, bevorzugt als arithmetisches Mittel oder Zentralwert (Median) ausgehend von den Temperaturmesswerten des im Schritt (b) ermittelten Zeitbereichs.

Bei der Temperaturmethode geht es darum im weiblichen Zyklus den Zeitpunkt des hormonell bedingten Temperatursprungs von Tief- zu Hochlage zu erkennen - dieser beträgt etwa 0.5°C.

Im menschlichen Körper gibt es auch innerhalb eines Tags - in Abhängigkeit von körperlicher Aktivität - Temperaturschwankungen, die 0.5°C übersteigen.

Die Temperaturmethode funktioniert also umso zuverlässiger, je weniger die jeweilig gemessene Körpertemperatur von nicht hormonell bedingten Einflüssen befreit ist. Daher wird klassischerweise per Fieberthermometer morgens nach der Nachtruhe immer zum selben Zeitpunkt gemessen.

Einer der diesbezüglichen relevanten Vorteile der vorliegenden Erfindung ist es, statt einer einzigen Messung nach der Nachtruhe, in gewissen Intervallen (z.B.: 5 Minuten) während der gesamten Nacht Temperaturmessungen vorzunehmen, um hieraus eine Temperaturmessreihe zu erstellen.

Die Qualität der Auswertung im Hinblick auf die Genauigkeit der Bestimmung des Ovulationszeitpunktes ist dann weiterhin abhängig von der jeweiligen Berechnungsmethode bzw. dem jeweiligen Auswertungsalgorithmus. Die Methode oder der Algorithmus haben die Aufgabe, aus der jeweiligen nächtlichen Messreihe einen repräsentativen Temperaturwert zu identifizieren, der dann in die Monats-/Zykluskurve zur Ermittlung des Ovulationszeitpunktes einfließt.

In der Literatur wird in diesem Zusammenhang häufig von der "Basaltemperatur" gesprochen - das ist die niedrigste Temperatur, die sich (z.B.) in dieser Schlafphase einstellt. - In unserer Praxis zeigt sich jedoch, dass die Definition der Basaltemperatur als nächtliches Temperaturminium nicht optimal ist, da es sich dabei nicht selten um einen "Ausreißer", also ein singuläres Minimum handelt.

Wie die Erfinder herausgefunden haben, erlaubt eine Fokussierung auf die Temperaturmesswerte in einem Zeitpunkt von ca. 30 Minuten vor dem Aufwachen eine verbesserte Bestimmung der Basaltemperatur. Das prinzipielle obengenannte Problem (Gefahr singulärer Extrema) wird aber damit nicht eliminiert.

Hier leistet das oben beschriebene dreischrittige Verfahren eine entscheidende Verbesserung, insofern in der nächtlichen Temperaturkurve einen Zeitbereich von etwa 20 Minuten Länge mit der geringsten Temperaturamplitude in der Zeit etwa 20 - 90 Minuten vor dem Aufwachen gesucht wird und von diesem Zeitbereich der Temperaturmittelwert als Basaltemperatur definiert wird.

Der theoretische Hintergrund ist der, dass a) eine geringe Temperaturamplitude auf geringe körperliche Aktivität in dieser Schlafphase hinweist und b) die Gefahr singulärer Extrema reduziert ist

In einem weiteren Aspekt stellt die Erfindung ein Verfahren zur Bestimmung der Basaltemperatur bereit, wobei das Verfahren die folgenden Schritte umfasst:
(a) Bereitstellen einer über Nacht während der Schlafphase aufgenommenen Temperaturmessreihe eines weiblichen menschlichen Körpers mit einem Messintervall von zwischen 2 und 10 Minuten, und bevorzugt von 5 Minuten, wobei diese Temperaturmessreihe bevorzugt mit einem erfindungsgemäßen Funkthermometer und/oder einem erfindungsgemäßen Verfahren erstellt worden ist;
(b) Aufteilung der Temperaturmessreihe in Segmente S1 , S2 , S3 ...SN zeitlich gleicher Länge, bevorzugt von 5 Minuten bis 60 Minuten Länge;
(c) Bildung der jeweiligen Temperaturmittelwerte M1 , M2 , M3 ,..MN für die Segmente S1 , S2 , S3 ...SN , ausgehend von den Temperaturmesswerten der jeweiligen Segmente bevorzugt als arithmetisches Mittel oder Zentralwert (Median);
(d) Durchführung der Schritte (b) bis (c) für weitere gemäß Schritt (a) in Folgenächten aufgenommene Temperaturmessreihen, die bevorzugt jede Nacht erstellt worden sind; wobei die Segmentdauer und die Berechnungsmethode des Mittelwertes beibehalten worden sind;
(e) Erstellung von N monatlichen Zykluskurven durch Auftragen der jeweiligen Temperaturmittelwerte M1 , M2 , M3 ,..MN über die Zeit;
(f) Ermittlung derjenigen monatlichen Zykluskurve, die von den N Zykluskurven im monatlichen Temperaturverlauf die geringste Standardabweichung oder Varianz zeigt;
wobei bevorzugt die nächtlichen Temperaturmessreihen vor der Segmentbildung in Schritt (b) unter Berücksichtigung ähnlicher Temperaturverlaufsmuster bzw. des circadianen Temperaturrhythmus zeitlich ausgerichtet werden, und die Lage der Segmente S1 , S2 , S3 ...SN , nicht nur von der eigentlichen Messzeit sondern auch von einer durch die ausgerichteten Messreihen definierten Startzeit bestimmt wird.

Wie der Erfinder herausgefunden hat liefert, das vorab definierte Verfahren noch bessere Ergebnisse. Hierbei werden die nächtlichen Temperaturkurven in Segmente zeitlich gleicher Länge (z.B. 5 - 60 Minuten) aufteilt und deren Temperaturmittelwert (z.B. in Form des arithmetischen Mittelwerts oder des Medians) ermittelt.

Eine Messreihe, die etwa in der Zeit zwischen 1:00 - 7:00 Uhr aufgezeichnet wurde, wird also in 6 - 724 Segmente "zerlegt", aus deren jeweiligen Temperaturmittelwert sich zunächst entsprechend 6 - 72 monatliche Zykluskurven ergeben. Durch geeignete Verfahren, wie beispielsweise durch Regression oder KI-Musterkennung, dy statistisch, syntaktisch oder per neuronalem Netz erfolgen kann, wird daraus diejenige Zykluskurve ermittelt, die den hormonell bedingten monatlichen Temperaturverlauf im Zyklus optimal repräsentiert.

Im Ergebnis "dämpft" bzw. eliminiert dieses Verfahren singuläre Extrema im monatlichen Temperaturverlauf und exponiert gleichzeitig die Signifikanz des hormonell bedingten Temperatursprungs zum Zeitpunkt der Ovulation. Verschiedene statistische Verfahren sind zur weiteren Verarbeitung im Hinblick auf Erkennen des Zeitpunktes der Ovulation geeignet (s. Regime Change Models, etc.)

Vorzugsweise werden die nächtlichen Temperaturkurven (vor der Segmentierung) z.B. unter Berücksichtigung ähnlicher Temperaturverlaufsmuster oder circadianer Rhythmik zeitlich ausgerichtet (sog. Alignment).

Ein solches Funkthermometer kann in vorteilhafter Weise für die unterschiedlichsten Messzwecke verwendet werden. So kann es in Geräten eingesetzt werden, bei denen eine genaue und einfache Temperaturmessung über einen langen Zeitraum notwendig ist.

So kann ein solches Thermometer bei dem Versand temperatursensitiver Güter (Lebensmittel, Pharmaka) als Datenlogger zum Nachweis einer ununterbrochenen Kühlkette eingesetzt werden.

In einer weiteren Anwendungsmöglichkeit kann das Funkthermometer auch in Gebäuden zur Erstellung eines vernetztes, "intelligentes" Heims ("smart home") verwendet werden und kann hierbei beispielsweise die Energiekosten weiter senken.

Weiterhin kann es in anderen biologischen Systemen eingesetzt werden, so beispielsweise zur Messung des Brutverhaltens von Vögeln.

### Definitionen

Unter einem konfigurierbaren Wert ist gemäß der Erfindung ein Wert zu verstehen, der frei konfiguriert werden kann, also im Rahmen des Verfahrens dann den jeweils in der Konfiguration gegebenen beliebig vorabgewählten Wert annehmen kann. Hierbei ist auch explizit eine dynamische Konfigurierbarkeit mit eingeschlossen. Dies bedeutet, dass die Konfiguration z.B. in Reaktion auf die vorliegenden Testbedingungen dynamisch an diese Testbedingungen angepasst werden kann. So kann beispielsweise eine intravaginale Messreihe erst zu einem späteren Zeitpunkt mit einer zeitlich engmaschigen Temperaturerfassung beginnen, wenn die früheren Messungen ergeben, dass bei der betreffenden Person die Basaltemperatur erst entsprechend spät erreicht wird.

Unter einer "im wesentlichen steifen" Kunststoffkapsel ist im Rahmen der Erfindung eine Kunststoffkapsel zu verstehen, bei der der Kunststoff eine Härte gemäß DIN ISO-2039-1:2003-6 von mindestens 75 MPa bevorzugt von mindestens 90 MPa und besonders bevorzugt von mindestens 95 MPa aufweist.

Eine "Körperöffnung" im Sinne der Erfindung ist eine Öffnung an einem Körper eines Tieres oder Menschen. Dies umfasst als eigentliche Körperöffnungen Körperhöhlen wie die Vagina, das Rektum, der Gehörgang oder der Mundraum aber auch Körpermulden wie die Achselhöhle oder die Kniekehle.

Gemäß der Erfindung ist unter dem Begriff "hermetisch verschlossen" eine Kapsel zu verstehen, die wasserdicht verschlossen ist. Damit wird verhindert, dass Körperausscheidungen und hierbei insbesondere Körperflüssigkeiten wie beispielsweise Schweiß, Urin oder Zervikalflüssigkeit in das Innere der Kapsel eindringen können.

Bluetooth Low Energy (auch Bluetooth LE, BLE oder Bluetooth Smart genannt) ist eine Funktechnik, mit der sich Geräte in einer Umgebung von etwa 10 Metern bis zu 500 m bei neueren Versionen vernetzen lassen. Im Vergleich zum "klassischen" Bluetooth weist Bluetooth Smart / BLE deutlich geringeren Stromverbrauch und geringere Kosten bei einem ähnlichen Kommunikationsbereich auf. Die entsprechenden Bluetooth-Versionen sind bisher unter der 4.x oder der 5.x Spezifikation im Gebrauch.

### Kurze Beschreibung der Abbildungen

Diese und andere Aspekte der Erfindung werden im Detail in den Abbildungen wie folgt gezeigt.
- Fig. 1: zeigt eine schematische Skizze eines erfindungsgemäßen netzunabhängigen Funkthermometers. Dargestellt ist das Funkthermometer in Aufsicht (oben links), in den beiden senkrecht zueinander stehenden Seitenansichten (unten links und unten rechts) und in perspektivischer Darstellung (oben rechts).
- Fig. 2: zeigt eine schematische Skizze der in dem netzunabhängigen Funkthermometer vorgesehenen elektronischen Komponenten. Dargestellt sind die Komponenten in Seitenansicht (links) und in perspektivischer Darstellung (rechts).
- Fig. 3: zeigt ein Flussdiagramm zur Darstellung des Messverfahrens.

### Detaillierte Beschreibung der Ausführungsbeispiele

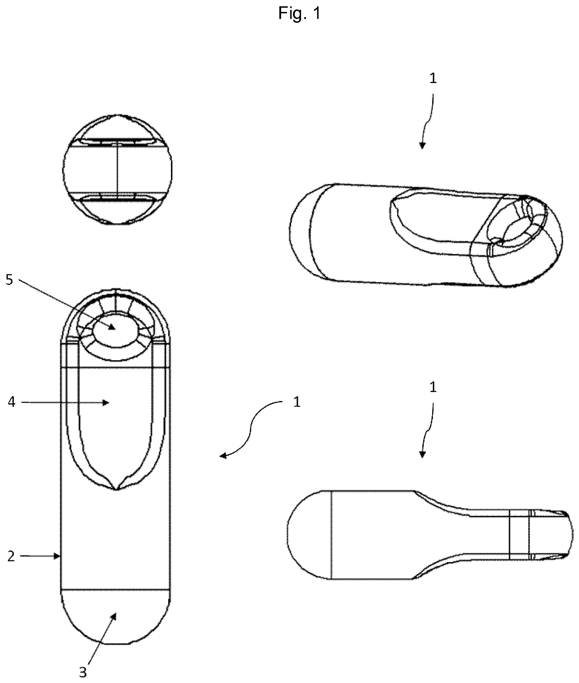

Fig. 1 zeigt eine schematische Skizze eines erfindungsgemäßen netzunabhängigen Funkthermometers (1) als zylinderförmiger Körper (2) mit halbkugelförmigen distalen Ende (3) zum einfachen Einführen in die Körperöffnung und einem abgeflachten Ende (4), das eine Öse (5) zur Aufnahme einer Zugvorrichtung (nicht dargestellt) aufweist.

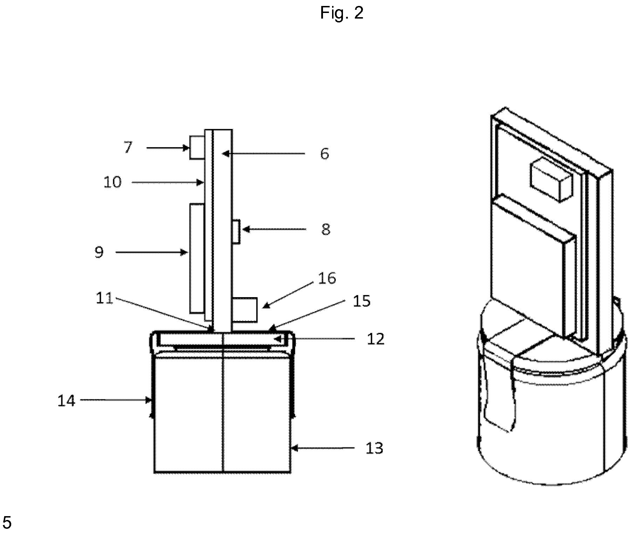

In der Fig. 2 werden die in der Kapsel des netzunabhängigen Funkthermometers vorgesehenen elektronischen Komponenten in ihrer Anordnung schematisch dargestellt. Hierbei ist eine erste Leiterplatte (6) mit einer Antenne (7), einem Temperatursensor (8), einem Kondensator (16), und mit einer Leiterplatte des BLE Moduls (9) mit Abschirmblech (9) bestückt und über eine Kante (11) an eine zweite kreisrunde Leiterplatte (12) gelötet. Diese zweite Leiterplatte steht mit dem Minuspol der Knopfzellenbatterie (13) in Verbindung, wobei die Verbindung zum Pluspol der Batterie über eine U-förmige Batteriekontaktfeder (14) hergestellt wird, deren mittlere Basis (15) zwischen erster und zweiter Leiterplatte angeordnet ist.

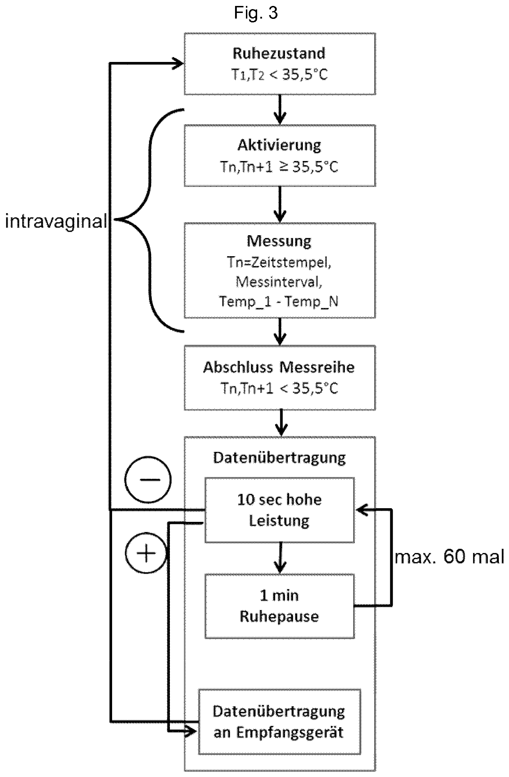

In der Fig. 3 werden die einzelnen Schritte des Mess- und Übertragungsverfahrens, wie in den Ansprüchen 11 und 13 beschrieben dargestellt. Im Teilschritt der Datenübertragung gibt es zwei alternative Folgeschritte. Bei erfolgreichem Verbindungsaufbau mit dem Empfangsgerät (Plus im Kreis) erfolgt im nächsten Schritt die Datenübertragung an das Empfangsgerät. Kann auch nach 60maliger Wiederholung keine Verbindung hergestellt werden (Minus im Kreis) so wird das Gerät wieder in den Ruhemodus versetzt.

Weitere Varianten der Erfindung und ihre Ausführung ergeben sich für den Fachmann aus der vorangegangenen Offenbarung, den Figuren und den Patentansprüchen.

In den Patentansprüchen verwendete Begriffe wie "umfassen", "aufweisen", "beinhalten", "enthalten" und dergleichen schließen weitere Elemente oder Schritte nicht aus. Die Verwendung des unbestimmten Artikels schließt eine Mehrzahl nicht aus. Eine einzelne Einrichtung kann die Funktionen mehrerer in den Patentansprüchen genannten Einheiten bzw. Einrichtungen ausführen. In den Patentansprüchen angegebene Bezugszeichen sind nicht als Beschränkungen der eingesetzten Mittel und Schritte anzusehen.

### Liste der Bezugszeichen

- 1: Netzunabhängiges Funkthermometer
- 2: Zylindrische Kapsel
- 3: Halbkugelförmiges distales Ende
- 4: abgeplattetes proximales Ende
- 5: Öse für Zugvorrichtung
- 6: erste Leiterplatte
- 7: Antenne
- 8: Temperatursensor
- 9: Abschirmblech des BLE-Moduls
- 10: Leiterplatte des BLE Moduls
- 11: Stromführende Kante der ersten Leiterplatte
- 12: Zweite Leiterplatte
- 13: Knopfzellenbatterie
- 14: Batteriekontaktfeder
- 15: Basis der Batteriekontaktfeder
- 16: Kondensator

## Patentansprüche

1. Netzunabhängiges Funkthermometer (1) zur Temperaturmessung in einer Körperöffnung für den Langzeiteinsatz mit einer hermetisch verschlossenen, in die Körperöffnung einführbaren, im Wesentlichen steifen Kunststoffkapsel enthaltend eine Knopfzellenbatterie (13) und als elektronische Komponenten:
(a) ein Single-Chip-Computer;
(b) einen Halbleiter-Temperatursensor (8);
(c) eine Kommunikationseinheit zur Signalübertragung;
(d) einen Datenspeicher;
wobei der Temperatursensor (8) und die Kommunikationseinheit über die Temperatur steuerbar sind,
so dass die Aktivierung des Funkthermometers (1) zum Aufnehmen einer Temperaturmessreihe ausgehend von einem Ruhezustand durch das Einbringen in die Körperöffnung und der dadurch hervorgerufenen Temperaturerhöhung auf einen physiologischen Wert induziert wird, und es die automatische Erkennung der Entnahme aus der Körperöffnung beinhaltet mit den beiden zentralen Folgen, dass 1.) die Messreihe abgeschlossen wird und 2.) eine Datenübertragung initiiert wird,
**dadurch gekennzeichnet, dass** die Kommunikationseinheit konfiguriert ist zur Signalübertragung nach dem BLE-Standard, und
das Funkthermometer (1) zu Erfassung, Speicherung und Weitergabe von inkrementellen oder dekrementellen Temperaturwerten ausgelegt ist, und
wobei die Inkremente oder Dekremente auf einen Basiswert oder auf der jeweils vorangehenden Wert bezogen werden.

2. Netzunabhängiges Funkthermometer (1) gemäß Anspruch 1, **dadurch gekennzeichnet, dass** die elektronischen Komponenten auf einer, bevorzugt beidseitig bestückten, ersten Leiterplatte (6) angebracht sind, wobei eine der Kanten der ersten Leiterplatte (6) als leicht gebogene metallisierte Kante direkt mit dem Minuspol der Knopfzellenbatterie (13) in Kontakt steht, und wobei die Verbindung zum Pluspol der Batterie über eine U-förmige Batteriekontaktfeder (14) hergestellt wird, deren mittlere Basis (15) bevorzugt in eine kantenseitig metallisierte Aussparung der Leiterplatte (6) eingreift.

3. Netzunabhängiges Funkthermometer (1) gemäß Anspruch 1 oder 2, **dadurch gekennzeichnet, dass** die Körperöffnung das Ohr, das Rektum oder die Vagina ist.

4. Netzunabhängiges Funkthermometer (1) gemäß Anspruch 1 bis 3, **dadurch gekennzeichnet, dass** das Funkthermometer (1) bei täglicher Erfassung einer Messreihe über mindestens 6 Stunden und täglicher Übermittlung der Daten einen Langzeiteinsatz über einen Zeitraum von mindestens einem Jahr, bevorzugt mindesten zwei Jahren und besonders bevorzugt von mindestens drei bis fünf Jahren ermöglicht.

5. Netzunabhängiges Funkthermometer (1) gemäß einem der vorherigen Ansprüche, **dadurch gekennzeichnet, dass** die Kunststoffkapsel aus einem Kapselgehäuse und einem Kapseldeckel besteht, die vor dem Einsatz als Thermometer durch Verklebung fest und wasserdicht miteinander verbunden werden, wobei für die Verklebung bevorzugt ein UV-härtender Cyanacrylatkleber eingesetzt wird.

6. Netzunabhängiges Funkthermometer (1) gemäß einem der vorherigen Ansprüche, **dadurch gekennzeichnet, dass** der Halbleiter-Temperatursensor (8) mindestens eine der folgenden Eigenschaften aufweist:
(a) Eine Auflösung von 0,05 °C im Bereich der Körpertemperatur;
(b) Abgabe einer digital umgesetzten Spannung über eine serielle Schnittstelle, die bevorzugt eine I 2 C-Schnittstelle ist;
(c) Eine Langzeitstabilität von weniger als 0,05°C/Jahr, bevorzugt von weniger als 0,01 °C/Jahr;
(d) Anwesenheit eines integrierten Mikrocontrollers, der die Messfehler des eigentlichen Halbleitersensors korrigiert;
(e) Fabrikseitige Kalibrierung mit Speicherung der Kalibrierdaten auf dem Temperatursensor-Chip in einem Permanentspeicher;
(f) ein durchschnittlicher Stromverbrauch von weniger als 200 nA pro Messung bei einer Messung pro Sekunde.

7. Netzunabhängiges Funkthermometer (1) gemäß einem der vorherigen Ansprüche, **dadurch gekennzeichnet, dass** die elektronischen Bauteile wie Datenspeicher, Bluetooth LE-Übertragungseinheit und Steuerungseinheit als Ein-Chip-System vorliegen.

8. Verfahren zur Messung der Basaltemperatur zur Ovulationsbestimmung umfassend die folgenden Schritte:
(a) Bereitstellen eines netzunabhängigen Funkthermometers (1) in einem Ruhemodus, das Folgendes umfasst: einen Single-Chip-Computer; einen Halbleiter-Temperatursensor (8); eine Kommunikationseinheit zur Signalübertragung nach dem BLE-Standard; einen Datenspeicher; eine Knopfzellenbatterie (13); wobei der Temperatursensor (8) und die Kommunikationseinheit über die Temperatur steuerbar sind, und wobei das Funkthermometer (1) zu Erfassung, Speicherung und Weitergabe von inkrementellen oder dekrementellen Temperaturwerten ausgelegt ist, wobei ein netzunabhängiges Funkthermometer (1) gemäß einem der Ansprüche 1 bis 7 bereitgestellt wird;
(b) Einführen des Funkthermometers (1) in eine Körperöffnung einer Frau, bevorzugt in die Vagina;
(c) Aufnehmen einer Temperaturmessreihe, bevorzugt über Nacht;
(d) Speicherung der Messdaten als inkrementelle oder dekrementelle Temperaturwerte in einem Speicher des Funkthermometers (1);
(e) Entnehmen des Funkthermometers (1) aus der Körperöffnung;
(f) Übertragen der Temperaturmessdaten als inkrementelle oder dekrementelle Temperaturwerte mittels Bluetooth Smart an ein Empfangsgerät, das bevorzugt ein Mobiltelefon, Tablet oder Computer ist;
(g) optionale Reinigung des Funkthermometers (1) und Lagerung außerhalb der Körperöffnung;
(h) Wiederholung der Schritte (b) bis (g), bevorzugt täglich, zur Erhebung einer Langzeitmessreihe zur Ermittlung relevanter Ereignisse im Menstruationszyklus wie Ovulationszeitpunkt, empfängnisfreie Phase oder fruchtbare Phase.

9. Verfahren gemäß Anspruch 8, **dadurch gekennzeichnet, dass** das Funkthermometer (1) im Ruhemodus gemäß Schritt (a) einen zeitgesteuerten Zähler inkrementiert und nach einer konfigurierbar festgelegten Zählzeit, die bevorzugt zwischen 10 Minuten und 60 Minuten, besonders bevorzugt zwischen 15 und 45 Minuten und insbesondere bevorzugt 30 Minuten beträgt, eine energiesparende Temperaturmessung mit geringer Präzision durchführt, und das Messergebnis mit dem Schwellenwert von zwischen 35 und 36°C vergleicht, wobei der Ruhemodus solange aufrechterhalten bleibt, wie nicht mindestens eine Anzahl von durch Konfiguration festgelegten aufeinanderfolgenden Messwerten, die bevorzugt mindestens zwei aufeinanderfolgende Messwerte sind, über dem Schwellenwert liegen.

10. Verfahren gemäß Anspruch 8 oder 9, **dadurch gekennzeichnet, dass** das Funkthermometer (1) zur Initialisierung der Messreihe in Schritt (c) bei einer konfigurierbaren, mindestens 2 betragenden Anzahl von aufeinanderfolgenden Messwerten über dem zwischen 35 und 36°C liegenden Schwellenwert Folgendes durchführt:
i. die Messintervalle reduziert;
ii. die Messauflösung erhöht und bevorzugt maximiert;
iii. die Messwerte als Messreihe abspeichert, wobei die Temperaturwerte als Inkremente ausgehend von einer Offset-Temperatur oder dem vorausgehenden Messwert gespeichert werden.

11. Verfahren gemäß einem der Ansprüche 8 bis 10, **dadurch gekennzeichnet, dass** die Speicherung der Daten in Schritt (d) in einem Ringpuffer implementiert ist, bei dem die ältesten Inhalte überschrieben werden, wenn der Puffer voll ist und weitere Elemente in den Ringpuffer abgelegt werden können.

12. Verfahren gemäß einem der Ansprüche 8 bis 11, **dadurch gekennzeichnet, dass** das Funkthermometer (1) nach Entnahme des Funkthermometers (1) in Schritt (e) und Detektion einer konfigurierbaren, mindestens 2 betragenden Anzahl von aufeinanderfolgenden Messwerten unter dem zwischen 35 und 36°C liegenden Schwellenwert die Messreihe abschließt und der Bluetooth-Übertragungseinheit die Verfügbarkeit einer abgeschlossenen Messreihe signalisiert.

13. Verfahren gemäß einem der Ansprüche 8 bis 12, **dadurch gekennzeichnet, dass** die Bluetooth-Übertragungseinheit in Schritt (f) die folgenden Verfahrensschritte durchführt:
i. die Übertragungseinheit versucht, für eine kurze Zeitspanne von weniger als 1 min bevorzugt weniger als 30 sec und besonders bevorzugt für 10 sec mit hoher Sendeleistung, eine Verbindung mit einer Empfangseinheit aufzubauen;
ii. bei erfolgreichem Verbindungsaufbau überträgt die Verbindungseinheit die abgeschlossene(n) Messreihe(n) an die Empfangseinheit und die entsprechenden Daten werden aus dem Speicher des Funkthermometers (1) gelöscht; oder
iii. bei erfolglosen Verbindungsaufbau wird für eine Zeitspanne von zwischen 30 s und 5 min, bevorzugt von zwischen 45 s und 2 min und insbesondere für 1 min die Sendeleistung der Übertragungseinheit reduziert oder ganz abgeschaltet,
iv. Iteration der Schritte i. und iii. mit einer konfigurierbaren Iterationsrate von bevorzugt zwischen 40 und 80 und besonders bevorzugt von 60;
v. bei nicht erfolgreichem Verbindungsaufbau nach Erreichen der konfigurierten Iterationsrate erfolgt das Abschalten der Sendeinheit bis zum Abschluss einer weiteren Messreihe.

14. Verfahren gemäß einem der Ansprüche 8 bis 13, **dadurch gekennzeichnet, dass** bei der Messreihe zur Angabe der jeweiligen Messzeitpunkte nur ein Messwert einen Zeitstempel enthält und die weiteren Zeitpunkte durch Abspeicherung des Messintervalls definiert sind.

## Claims

1. Off-line radio thermometer (1) for temperature measurement in a body opening for long-term use with a hermetically sealed, into the body opening insertable, essentially stiff plastic capsule, containing a coin cell battery (13) and as electronic components:
(a) a single-chip computer;
(b) a semiconductor temperature sensor (8);
(c) a communication unit for signal transmission;
(d) a data storage;
where the temperature sensor (8) and the communication unit can be controlled via the temperature,
so that the activation of the radio thermometer (1) for recording a series of temperature measurements is induced starting from a idle state by the insertion into the body opening and the resulting temperature increase to a physiological value, and involves the automatic detection of the extraction from the body opening with the two central consequences that 1.) the measurement series is completed and 2.) a data transmission is initiated,
**characterized in that**
the communication unit is configured for signal transmission according to the BLE standard, and the radio thermometer (1) designed is for measuring, storing and transmitting of incremental or decremental temperature values, and where the increments or decrements are related to a base value or to the immediately preceding value.

2. Off-line radio thermometer (1) according to claim 1, **characterized in that** the electronic components are mounted on a - preferably on both sides populated - first printed circuit board (6), whereby one of the edges of the first printed circuit board (6) has direct contact with the negative terminal of the coin cell battery (13) as a slightly curved metallized edge, and the connection to the positive terminal of the battery is established via a U-shaped battery contact spring (14), the central base (15) of which preferably engages in a cutout in the printed circuit board (6) which is metallized on the edge side.

3. Off-line radio thermometer (1) according to claim 1 or 2, **characterized in that** the body opening is the ear, the rectum or the vagina.

4. Off-line radio thermometer (1) according to claim 1 to 3, **characterized in that** with daily recordings of a series of measurements over at least 6 hours and daily transmissions of the data, the radio thermometer (1) enables long-term use over a period of at least one year, preferably of at least two years and particularly preferably of at least three to five years.

5. Off-line radio thermometer (1) according to one of the preceding claims, **characterized in that** the plastic capsule consists of a capsule housing and a capsule lid, which are firmly and water-tightly connected to one another by gluing before being used as thermometers, wherein a UV-curing cyanoacrylate adhesive is preferably used for the bonding.

6. Off-line radio thermometer (1) according to one of the preceding claims, **characterized in that** the semiconductor temperature sensor (8) has at least one of the following properties:
(a) a resolution of 0.05° C. in the body temperature range;
(b) delivery of a digitally converted voltage via a serial interface, which is preferably an I2C interface;
(c) a long-term stability of less than 0.05° C./year, preferably less than 0.01° C./year;
(d) presence of an integrated microcontroller that corrects errors the measurement of the actual semiconductor sensor;
(e) factory-side calibration with storage of the calibrator data on the temperature sensor chip in a permanent storage;
(f) an average power consumption of less than 200 nA per measurement at at one measurement per second.

7. Off-line radio thermometer (1) according to one of the preceding claims, **characterized in that** the electronic components such as data storage, Bluetooth LE transmission unit and control unit are present as a single-chip system.

8. Method for measuring the basal temperature for ovulation determination, which comprises the following steps:
(a) providing an off-line radio thermometer (1) in a idle mode, comprising: a single-chip computer; a semiconductor temperature sensor (8); a communication unit for signal transmission according to the BLE standard; a data storage; a coin cell battery (13); wherein the temperature sensor (8) and the communication unit are controllable via temperature, and wherein the radio thermometer (1) is designed for recording, storing and transmitting incremental or decremental temperature values, providing an off-line radio thermometer (1) in accordance with one of claims 1 to 7;
(b) inserting the radio thermometer (1) into a woman's body opening, preferably into the vagina;
(c) recording a series of temperature measurements, preferably overnight;
(d) storing of the measurement data as incremental or decremental temperature values in a storage of the radio thermometer (1);
(e) taking the radio thermometer (1) out of the body opening;
(f) transmitting the temperature measurement data as incremental or decremental temperature values by means of Bluetooth Smart to a receiving device which is preferably a mobile phone, tablet or computer;
(g) optional cleaning of the radio thermometer (1) and storage outside the body opening;
(h) repeating of steps (b) to (g), preferably daily, for the collection of a long-term measurement series to determine relevant events in the menstrual cycle such as ovulation time, non-conception phase or fertile phase.

9. Method according to claim 8, **characterized in that** the radio thermometer (1) in idle mode increments a time-controlled counter according to step (a) and, after a configurable set counting time, which is preferably between 10 minutes and 60 minutes, particularly preferably between 15 and 45 minutes and especially preferably 30 minutes, performs an energy-saving temperature measurement with low precision and compares the measurement result with the threshold value between 35 and 36°C, where the idle mode is maintained as long as at least a number of consecutive measurements, defined by configuration, preferably at least two consecutive measurements, are above the threshold value.

10. Method according to claim 8 or 9, **characterized in that** the radio thermometer (1) for initializing the measurement series in step (c), performs after a configurable number of at least 2 consecutive measured values above the threshold value between 35° and 36° C. the following steps:
I. it reduces the measurement intervals;
li. it increases and preferably maximizes the measurement resolution;
lii. it stores the measured values as a measurement series, whereby the temperature values are stored as increments starting from an offset temperature or the preceding measured value.

11. Method according to one of claims 8 to 10, **characterized in that** the storage of the data in step (d) is implemented as a ring buffer, in which the oldest contents are overwritten when the buffer is full so that further elements can be stored in the ring buffer.

12. Method according to one of claims 8 to 11, **characterized in that** the radio thermometer (1), after removal of the radio thermometer (1) in step (e) and detection of a configurable number of at least 2 consecutively measured values below the threshold value lying between 35 and 36°C, completes the series of measurements and signals the availability of a completed series of measurements to the Bluetooth transmission unit.

13. Method according to one of claims 8 to 12, **characterized in that** the Bluetooth transmission unit in step (f) performs the following methodic steps:
i. the transmission unit attempts to establish a connection with a receiving unit for a short period of time of less than 1 min preferably less than 30 sec and particularly preferably for 10 sec with high transmission power;
ii. if the connection is successfully established, the connection unit transmits the completed series(es) of measurements to the receiving unit and the corresponding data are deleted from the memory of the radio thermometer (1); or
iii. if the connection is unsuccessful, the transmission power of the transmission unit is reduced or switched off completely for a period of between 30 s and 5 min, preferably between 45 s and 2 min and in particular for 1 min,
iv. Iteration of steps i. and iii. with a configurable iteration rate of preferably between 40 and 80 and particularly preferably 60;
v. if the connection is not successfully established after the configured iteration rate is reached, the transmission unit is switched off until a further series of measurements has been completed.

14. Method according to one of claims 8 to 13, **characterized in that**, for specifying the respective measurement times, only one measurement value of the measurement series contains a time stamp and the other times are defined by storing the measurement interval.

## Revendications

1. Thermomètre radio sans réseau (1) pour la mesure de la température dans une ouverture du corps pour une utilisation à long terme avec une capsule en plastique hermétiquement fermée, insérable dans l'ouverture du corps, essentiellement rigide, contenant une pile bouton (13) et comprenant les composants électroniques suivants:
(a) un ordinateur à puce unique;
(b) un capteur de température à semi-conducteur (8);
(c) une unité de communication pour la transmission d'un signal;
(d) une mémoire de données;
où le capteur de température (8) et l'unité de communication peuvent être commandés par la température,
de sorte que l'activation du thermomètre radio (1) pour l'enregistrement d'une série de mesures de température est induite à partir d'un état de repos par l'introduction dans l'ouverture corporelle
et l'augmentation de température ainsi provoquée jusqu'à une valeur physiologique, et il implique la reconnaissance automatique du prélèvement de l'ouverture du corps avec les deux conséquences centrales que 1.) la série de mesures est terminée et que 2.) une transmission de données est initiée, **caractérisé en ce que** l'unité de communication est configurée pour la transmission du signal selon la norme BLE et le thermomètre radio (1) est conçu pour enregistrer, stocker et transmettre des valeurs de température incrémentielles ou décrémentelles
où les incréments ou décréments sont rapportés à une valeur de base ou respectivement à la valeur précédente.

2. Thermomètre radio sans réseau (1) selon la revendication 1, **caractérisé en ce que** les composants électroniques sont montés sur une première carte de circuit imprimé (6), de préférence peuplée des deux côtés, dans laquelle l'un des bords de la première carte de circuit imprimé (6) est en contact direct avec le terminal négatif de la pile bouton (13) sous la forme d'un bord métallisé légèrement incurvé, et que la connexion à la borne positive de la batterie est établie par l'intermédiaire d'un ressort de contact de batterie en forme de U (14), dont la base centrale (15) s'engage de préférence dans une découpe de la carte de circuit imprimé (6) qui est métallisée sur son bord.

3. Thermomètre radio sans réseau (1) selon la revendication 1 ou 2, **caractérisé en ce que** l'ouverture du corps est l'oreille, le rectum ou le vagin.

4. Thermomètre radio sans réseau (1) selon les revendications 1 à 3, **caractérisé en ce que**, le thermomètre radio (1) permet, en cas de saisie quotidienne d'une série de mesures sur au moins 6 heures et de transmission quotidienne des données, une utilisation à long terme sur une période d'au moins un an, de préférence d'au moins deux ans et de manière particulièrement préférée d'au moins trois à cinq ans.

5. Thermomètre radio sans réseau (1) selon l'une des revendications précédentes, **caractérisé en ce que** la capsule en plastique se compose d'un boîtier de capsule et d'un couvercle de capsule, qui sont reliés ensemble de manière solide et imperméable avant d'être utilisés comme thermomètre par collage, avec de préférence une colle cyanacrylate à durcissement UV pour le collage.

6. Thermomètre radio sans réseau (1) selon l'une des revendications précédentes, **caractérisé en ce que** le capteur de température à semi-conducteur (8) présente au moins l'une des caractéristiques suivantes :
(a) une résolution de 0,05 °C dans la plage de température corporelle ;
(b) l'émission d'une tension convertie numériquement via une interface série, qui est de préférence une interface I²C ;
(c) une stabilité à long terme de moins de 0,05 °C/an, de préférence de moins de 0,01 °C/an
(d) la présence d'un microcontrôleur intégré qui corrige les erreurs de mesure du capteur à semi-conducteur proprement dit ;
(e) l'étalonnage en usine avec stockage des données d'étalonnage sur la puce du capteur de température dans une mémoire permanente ;
(f) une consommation d'énergie moyenne inférieure à 200 nA par mesure pour une mesure par seconde.

7. Thermomètre radio sans réseau (1) selon l'une des revendications précédentes, **caractérisé en ce que** les composants électroniques tels que le stockage de données, l'unité de transmission Bluetooth LE et l'unité de commande sont présents sous la forme d'un système intégré sur une seule puce.

8. Procédé de mesure de la température basale pour la détermination de l'ovulation comprenant les étapes suivantes:
(a) la mise à disposition d'un thermomètre radio sans réseau (1) en mode repos comprenant: un ordinateur à puce unique, un capteur de température à semi-conducteur (8); une unité de communication pour la transmission de signaux selon la norme BLE; une mémoire de données; une pile bouton (13); le capteur de température (8) et l'unité de communication étant contrôlables par la température, et où le thermomètre radio (1) est conçu pour enregistrer, stocker et transmettre des valeurs de température incrémentales ou décrémentales, un thermomètre radio sans réseau (1) étant fourni conformément à l'une des revendications 1 à 7;
(b) insertion du thermomètre radio (1) dans l'ouverture du corps d'une femme, de préférence dans le vagin.
(c) enregistrement d'une série de mesures de température, de préférence pendant la nuit;
(d) stockage des données de mesure sous forme de valeurs de température incrémentielles ou décrémentelles dans une zone de mémoire du thermomètre radio (1);
(e) retrait du thermomètre radio (1) de l'ouverture du corps;
(f) transmission des données de mesure de la température sous forme de valeurs de température incrémentielles ou décrémentelles à l'aide de Bluetooth Smart à un appareil récepteur, de préférence un téléphone portable, une tablette ou un ordinateur ;
(g) nettoyage facultatif du thermomètre radio (1) et stockage à l'extérieur de l'ouverture du corps;
(h) la répétition des étapes (b) à (g), de préférence quotidiennement, pour l'enquêter sur une série de mesures à long terme pour déterminer les événements pertinents du cycle menstruel tels que le moment de l'ovulation, la phase sans conception ou la phase fertile.

9. Procédé selon la revendication 8, **caractérisé par le fait que** le thermomètre radio (1) incrémente un compteur chronométré en mode veille conformément à l'étape (a) et effectue une mesure de température économe en énergie avec une faible précision après un temps de comptage configurable, qui est de préférence entre 10 minutes et 60 minutes, de préférence entre 15 et 45 minutes et en particulier de préférence 30 minutes, et compare le résultat de mesure avec la valeur seuil comprise entre 35 et 36°C, le mode veille étant maintenu tant qu'un nombre de mesures consécutives déterminé par configuration, qui sont de préférence au moins deux valeurs de mesure consécutives, n'est pas supérieur au seuil.

10. Procédé selon la revendication 8 ou 9, **caractérisé en ce que** le thermomètre radio (1) pour l'initialisation de la série de mesure à l'étape (c) effectue ce qui suit pour un nombre configurable d'au moins 2 valeurs de mesure successives au-dessus de la valeur de seuil comprise entre 35 et 36°C:
i. réduit les intervalles de mesure;
ii. augmente et maximise de préférence la résolution de mesure;
iii. enregistre les valeurs de mesure en tant que série de mesures, les valeurs de température étant enregistrées sous forme d'incréments à partir d'une température de décalage ou de la valeur mesurée précédente.

11. Procédé selon l'une des revendications 8 à 10, **caractérisé en ce que** le stockage des données à l'étape (d) est implémenté dans un tampon annulaire, dans lequel les contenus les plus anciens sont écrasés lorsque le tampon est plein et que d'autres éléments peuvent être déposés dans le tampon annulaire.

12. Procédé selon l'une des revendications 8 à 11, **caractérisé en ce que** le thermomètre radio (1), après avoir retiré le thermomètre radio (1), à l'étape (e) et lors de la détection, d'un nombre configurable d'au moins 2 de valeurs de mesure successives sous la valeur de seuil comprise entre 35 et 36°C, termine la série de mesures et signale à l'unité de transmission Bluetooth la disponibilité d'une série de mesures achevée.

13. Procédé selon l'une des revendications 8 à 12, **caractérisé en ce que** l'unité de transmission Bluetooth effectue les étapes de procédure suivantes à l'étape (f) :
i. l'unité de transmission tente, pendant une courte période de moins de 1 min, de préférence à moins de 30 sec et de préférence pour 10 sec à haute puissance d'émission, d'établir une connexion avec une unité de réception ;
ii. En cas d'établissement réussi de la connexion, l'unité de connexion transmet la ou les séries de mesures terminées à l'unité de réception et les données correspondantes sont effacées de la mémoire du thermomètre radio (1); ou
iii. En cas d'établissement infructueux de la connexion, la puissance d'émission de l'unité de transmission est réduite ou complètement éteinte pendant une période comprise entre 30 s et 5 min, de préférence entre 45 s et 2 min et en particulier pendant 1 min,
iv. Itération des étapes i. et iil avec un taux d'itération configurable de préférence entre 40 et 80 et particulièrement de 60;
v. En cas d'échec de l'établissement de la connexion après avoir atteint le taux d'itération configuré, l'unité d'émission est désactivée jusqu'à la fin d'une autre série de mesures.

14. Procédé selon l'une des revendications 8 à 13, **caractérisé en ce que**, pour spécifier les temps de mesure respectifs, une seule valeur de mesure de la série de mesures contient un horodatage et les autres temps sont définis par le stockage de l'intervalle de mesure.
